(12) # EUROPEAN PATENT SPECIFICATION

(45) Date of publication and mention of the grant of the patent:
**06.10.2021 Bulletin 2021/40**

(21) Application number: **16897630.6**

(22) Date of filing: **06.05.2016**

(51) Int Cl.:
*C12N 5/0783* *(2010.01)*          *A61K 35/17* *(2015.01)*
*A61P 35/00* *(2006.01)*

(86) International application number:
**PCT/CN2016/081198**

(87) International publication number:
**WO 2017/173696 (12.10.2017 Gazette 2017/41)**

(54) **EX VIVO EXPANSION METHOD FOR CORD BLOOD NK CELL, AND KIT AND APPLICATION THEREOF**

EX-VIVO-EXPANSIONSVERFAHREN FÜR NK-ZELLEN AUS NABELSCHNURBLUT SOWIE KIT UND VERWENDUNG DAVON

PROCÉDÉ D'EXPANSION EX VIVO DESTINÉ AUX CELLULES NK DU SANG OMBILICAL, ET TROUSSE ET APPLICATION ASSOCIÉES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **07.04.2016 CN 201610214351**

(43) Date of publication of application:
**13.02.2019 Bulletin 2019/07**

(73) Proprietor: **Beijing Jingmeng Stem Cell Technology Co., Ltd.**
**Beijing 100085 (CN)**

(72) Inventors:
• **WU, Xiaoyun**
**Beijing 100085 (CN)**
• **LIU, Xuemin**
**Beijing 100085 (CN)**
• **KANG, Huiyan**
**Beijing 100085 (CN)**
• **WANG, Yunhong**
**Beijing 100085 (CN)**
• **FAN, Xiaoxiang**
**Beijing 100085 (CN)**
• **LI, Zhigang**
**Beijing 100085 (CN)**
• **DING, Wei**
**Beijing 100085 (CN)**
• **WU, Lihua**
**Beijing 100085 (CN)**
• **LI, Jinyan**
**Beijing 100085 (CN)**
• **SHEN, Dianfu**
**Beijing 100085 (CN)**
• **ZHANG, Huizhen**
**Beijing 100085 (CN)**

(74) Representative: **Petculescu, Ana-Maria Bayramoglu Law Offices LLC Türkiye Irtibat Ofisi, Mira Office, Kanuni Sultan Süleyman Boulevard, 5387. street Beytepe, floor 12, no:50 06800 Cankaya, Ankara (TR)**

(56) References cited:
WO-A1-2011/080740          WO-A1-2012/009422
WO-A1-2014/053650          WO-A2-2014/089169
CN-A- 105 112 370          CN-A- 105 462 923
US-A1- 2016 075 996

• JAN SPANHOLTZ ET AL: "Clinical-Grade Generation of Active NK Cells from Cord Blood Hematopoietic Progenitor Cells for Immunotherapy Using a Closed-System Culture Process", PLOS ONE, vol. 6, no. 6, 16 June 2011 (2011-06-16), page e20740, XP055014138, ISSN: 1932-6203, DOI: 10.1371/journal.pone.0020740

**Description**

FIELD OF THE INVENTION

The present invention relates to the field of cell engineering technology, in particular to an ex vivo expansion method for cord blood NK cells.

BACKGROUND OF THE INVENTION

Natural killer (NK) cells are a third type of lymphocytes in addition to T cells and B cells, and they also have different cell morphology from the T cells and B cells. The NK cells are large granular lymphocytes in cytoplasm and containing azurophilic granules, and are widely found in lymphoid organs and peripheral tissues. The NK cells account for 5% to 10% of the total number of lymphocytes in normal peripheral blood, account for 1% to 2% of the total number of lymphocytes in the spleen, and also exist in lymph nodes and bone marrow. As an important constituent part of the human body's natural immunity, the NK cells are important immunoregulatory cells for the organism to resist infection and prevent malignant transformation of cells, and play a very important role in immune surveillance and immune defense. The past studies show that the proportion, number and function of the NK cells may be reduced in the development of certain diseases, such as tumors, autoimmune diseases, aging-related diseases and HIV infection or the like. The US Disease Control Center reported in 2012 that the vitality and number of NK cells are important factors for ensuring human body health. The onsets of almost all diseases are related to apparent insufficiency of vitality of the NK cells. When people have a disease, either chronic or sporadic or acute, the vitality of the NK cells is below an average level. A good disease treatment is to recover the NK cells to a high vitality level. Different from the T lymphocytes, the NK cells have the following characteristics. The NK cells can directly lyse and destroy tumor cells and virus-infected cells without pre-stimulation. Cell membranes are degraded by secreting perforin, serine proteases such as granzymes A and B, and molecules such as chondroitin sulfate proteoglycans, so as to destroy the integrity of target cells and achieve a cytolytic effect. Apoptosis of the target cells may be caused when NK cell surface expression Fas ligand (FasL) and TNF-related apoptosis-inducing ligand (TRAIL) bind with receptors on the target cells. Activated NK cells will release a large number of cytokines such as interferon-y and tumor necrosis factor-$\alpha$. The NK cells bind with an Fc segment of an antibody via cell membrane FcyRIII (CD16) to mediate antibody-dependent cytotoxicity. With the understanding of the biological function and activation mechanism of the NK cells, an immunotherapy using the NK cells as target points is of importance to an early prevention, control and treatment of diseases.

In recent years, immunotherapy has become the fourth largest treatment mode after surgery, radiotherapy-chemotherapy and endocrine therapy, and has gradually received more and more attentions. Adoptive cellular immunotherapy, as one of the immunotherapy methods, has received major developments in clinical studies. Currently, clinical studies have been carried out, in which autologous NK cells are applied to hematological tumors (acute myeloid leukemia, acute lymphocytic leukemia, etc.) and solid tumors (glioma, renal cancer and malignant melanoma) by adoptive immunotherapy. The clinical studies are confirmed to be safe, but the results of clinical trials are not so satisfactory. Specifically, by reinfusing autologous peripheral blood after an NK cell sorting is performed on the autologous peripheral blood, the survival rate and recurrence rate of patients are not improved as compared with a control group, although it is observed that the killing capability of the NK cells on tumor cells is improved, which may be related to an immune evasion prone to being formed for the autologous NK cells during the development of the tumor. In addition, subjects for the clinical trials of the NK cell adoptive immunotherapy are often extremely advanced patients who have experienced various chemotherapy failures, have a heavy tumor burden, a poor organism immunity, and NK cells with serious deficient functions.

To improve the efficacy of clinical treatment of the NK cells, it is necessary to change the previous treatment strategy. In order to overcome these defects, NK cells of an allogeneic origin are good choice. It is discovered by some scholars via research that the recurrence of leukemia can be delayed without causing graft-versus-host disease if leukemia patients are treated by transplanting allogeneic NK cells, and this has drawn people's attention to allogeneic reactivity of NK cells. It can be seen from the results of current clinical studies, heterologous NK cells are significantly superior to autologous NK cells in terms of complete remission rate, event-free survival, recurrence rate, and mortality. Furthermore, the heterologous NK cells are not accompanied by graft-versus-host reaction. The killing ability of NK cells to tumor cells depends on a regulation of signal balance between NK cell surface activating receptors and NK cell surface inhibitory receptors. Killer cell immunoglobulin-like receptors (KIR) form a receptor family which binds with major histocompatibility class I (MHC-I), and play an important role in regulating the activation threshold of human NK cells. Recently, it is shown by more and more studies that the killing ability of the NK cells to receptor tumor cells and the effect of NK cell adoptive immunotherapy both can be greatly improved by screening an optimum donor when using the mismatch of KIR receptor/MHC-I ligand. Currently, the KIR-based donor selection has received great attention in improving hematopoietic stem cell/bone marrow transplantation and adoptive immunotherapy for tumors. The heterologous NK infusion has become a new strategy for clinical NK cell adoptive immunotherapy.

Cord blood, as a ready-made heterologous resource, has no ethical problem and has rich sources, and it is easy to find the optimum donor of the cord blood. In addition, T lymphocytes in the cord blood develop immaturely and most of them are juvenile cells. Hence, after a transplantation, acute and chronic graft-versus-host diseases have a low probability of occurrence and a light degree of severity, so the cord blood has become a very promising source of heterologous NK cells. For a clinical infusion of NK cells, a sufficient number of cells with a sufficient purity are required. However, the NK cells account for a small proportion of cord blood cells, and the number of the NK cells is small. For clinical application, a large quantity of high-purity NK cells have to be obtained via ex vivo separation and culturing expansion. Therefore, how to obtain high-purity, high-quality NK cells has become the key issue of the NK cell adoptive immunotherapy (Document 1: Klingemann H: Challenges of cancer therapy with natural killer cells, Cytotherapy 2015, 17(3): 245-249.).

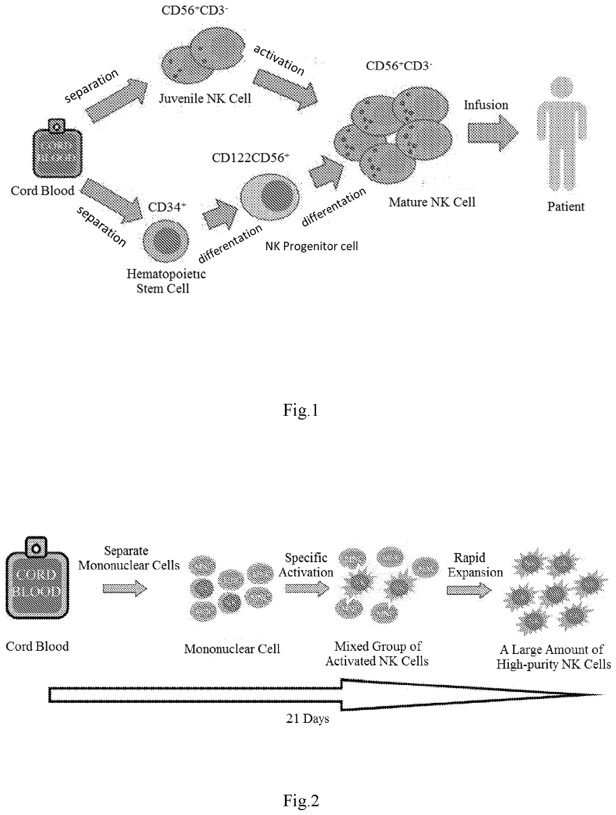

At present, there are mainly two methods for separating and expanding cord blood NK cells (as shown in FIG. 1). In method 1, the NK cells account for about 10% to 20% of lymphocytes in the cord blood, have immature functions and have a poor ability of killing tumor cells. The NK cells can be sorted and enriched by using a flow cytometer or a magnetic bead sorter. Then, the number of NK cells is increased and the ability of the NK cells to kill tumors is improved by ex vivo expansion. The Celgene Cellular Therapeutics Company separates mononuclear cells from frozen resuscitation cord blood by using lymphocyte separation solution, and sorts $CD56^+CD3^-$ NK cells by using the EasySep®NK enrichment kit (StemCell Technologies, the kit containing CD3, CD4, CD14, CD19, CD20, CD36, CD66b, CD123, HLA-DR and glycophorin A antibodies); the purity of the sorted cells can be up to 71%, and the number of the cells per part of cord blood is $1.5 \times 10^7$. Then, trophoblast cells (mitomycin C-treated peripheral blood mononuclear cells and K562 cells) are added via a starting culture medium (including: Iscoves modified Dulbecco medium (IMDM), 10% fatal bovine serum (FBS), 35mg/mL transferrin, 5$\mu$g/mL insulin, 20$\mu$M ethanolamine, 1$\mu$g/mL unsaturated fatty acid, 1$\mu$g/mL linoleic acid, 0.2$\mu$g/mL palmitic acid, 2.5$\mu$g/mL bovine serum albumin, 0.1$\mu$g/mL plant lectin, 1% cyan-streptomycin and 200IU/mL interleukin-2). After being cultured under a condition of 37°C and 5% $CO_2$ for 5-7 days, the NK cells are transferred to a maintenance culture medium (IMDM, 10% FBS, 2% human AB serum, 1% cyan-streptomycin, 200IU/mL interleukin-2) and cultured for 21 days. Then, $CD56^+CD3^-$ NK cells may be obtained for each part of cord blood, with a cell purity greater than 80% and an average total number of cells being $1.2 \times 10^9$ per part of cord blood (Document 2: Kang L, Voskinarian-Berse V, Law E, Reddin T, Bhatia M, Hariri A, Ning Y, Dong D, Maguire T, Yarmush M et al: Characterization and ex vivo Expansion of Human Placenta-Derived Natural Killer Cells for Cancer Immunotherapy. Frontiers in immunology 2013, 4:101.). In method 2, NK cells start from hematopoietic stem cells. The cord blood is rich in hematopoietic stem cells. The hematopoietic stem cells are sorted and enriched by using a flow cytometer or a magnetic bead sorter, and a certain number of mature NK cells are obtained by ex vivo differentiation. The Glycostem Therapeutics Company sorts and enriches $CD34^+$ hematopoietic stem cells with a purity of 67% and the number of $3.8 \times 10^6$ per part of cord blood, from frozen resuscitation cord blood by using a CliniMACS CD34 magnetic bead sorting kit. The cells are cultured for 9 days in cell expansion culture medium I (including: GBGM® culture medium, 10% human serum, high-dose cytokine combination (SCF, Flt3L, TP0, IL-7, low-molecular-weight heparin) and low-dose factor combination (GM-CSF, G-CSF, IL-6). Then, the cells are expanded for 14 days in cell expansion culture medium II (TP0 is replaced with IL-15, and other components are the same as the culture medium I), and finally, the cells are differentiated and cultured for 35 days in an NK cell differentiation culture medium (low-dose factor combination is replaced with (IL-7, SCF, IL-15 and IL-2), and others are the same as the culture medium I), to obtain NK cells with a purity greater than 90% and an average number of NK cells of $2 \times 10^9$ per part of the cord blood (Document 3: Spanholtz J, Preijers F, Tordoir M, Trilsbeek C, Paardekooper J, de Witte T, Schaap N, Dolstra H: Clinical-grade generation of active NK cells from cord blood hematopoietic progenitor cells for immunotherapy using a closed-system culture process, PloS one 2011, 6 (6): e20740.).

International Patent application WO2011080740 discloses methods of ex-vivo culture of natural killer (NK) cells, more particularly, methods for enhancing propagation and/or functionality of NK cells by treating the cells with a nicotinamide or other nicotinamide moiety in combination with cytokines driving NK cell proliferation. The use of IL-2 is disclosed.

International Patent application WO2012009422 also discloses methods for expanding NK-cells from cord blood, using IL-2.

Jan Spanholtz et al: "Clinical-grade Generation of active NK-cells from Cord Blood Hematopoietic Progenitor Cells for Immunotherapy Using a Closed-System Culture Process", PLOS ONE, vol. 6, 16 June 2011, Page e20740 describes the production of clinical-grade NK-cells populations from cord blood.

United States Patent application US2016075996 discloses a production method of BI-Cells enriched preparation. using an amount of IL-2 in the range 100 UImL to 2000U/mL, preferably 700 U/mL to 2000U/mL.

In summary, the methods of preparing cord blood NK cells in the prior art have the following disadvantages.

1: cells (NK cells or hematopoietic stem cells) have to be sorted, the sorting technology affects the activity of the NK cells, and a sorter is also required.

2: trophoblast cells are required, and an introduction of K562 cells greatly affects the safety of expanding the NK cells.

3: the operation is complicated and the technical versatility is poor.

4: the costs for separation stage and expansion stage are high.

5: the safety is poor due to the use of animal-source composition additives.

6: the yield of NK cells is low.

SUMMARY OF THE INVENTION

A first object of the present invention is to provide a simple and safe ex vivo expansion method for cord blood NK cells.

The ex vivo expansion method for cord blood NK cells according to the present invention includes:

step 1): activatedly culturing the cord blood NK cells by: adjusting the density of cord blood mononuclear cells to $0.5 \times 10^6$ to $5 \times 10^6$/mL using a lymphocyte culture medium, adding at least zoledronic acid with the concentration ranging from $1 \mu g$/mL to $10 \mu g$/mL and recombinant human interleukin-2 with the concentration ranging from 200IU/mL to 2000IU/mL, culturing for 1 to 5 days in an environment with a temperature ranging from 36°C to 40°C and $CO_2$ saturated humidity of 5%, and collecting cell fluid; and

step 2): proliferatedly culturing the cord blood NK cells by: obtaining cells from the cell fluid collected in step 1), adjusting the density of the cells to $0.5 \times 10^6$ to $5 \times 10^6$/mL using a lymphocyte culture medium, adding recombinant human interleukin-2 with the concentration ranging from 200IU/mL to 2000IU/mL, culturing in an environment with a temperature ranging from 36°C to 38°C and $CO_2$ saturated humidity of 5%, replenishing with fresh culture medium every other 2 to 3 days and adjusting the density of the cells to $0.5 \times 10^6$ to $5 \times 10^6$/mL, and culturing for 14 to 35 days to harvest the cord blood NK cells.

The cord blood mononuclear cells used in step 1) are separated from fresh anticoagulation cord blood or frozen resuscitation cord blood by:

1.1: taking the fresh anticoagulation cord blood or frozen resuscitation cord blood, and diluting it with PBS of a volume which is 1 to 2 times the volume of the cord blood;

1.2: adding slowly the diluted cord blood from above to the lymphocyte separation solution, and maintaining a clear interface between the diluted cord blood and the lymphocyte separation solution, wherein the volume of the diluted cord blood is equal to the volume of the lymphocyte separation solution;

1.3: centrifuging for 20 to 30 minutes at a room temperature and with a centrifugal force of 980g; and

1.4: after the centrifugation, aspirating a white membrane-like mononuclear cell layer, which is a second layer from the top, and washing the mononuclear cell layer with the PBS, to obtain cord blood mononuclear cells.

The lymphocyte culture medium used in step 1) may be for example AIM V® Medium CTS™ (commercially available from Life Technology Company, USA) or GMP S&XFM™-CD lymphocyte culture medium (the same name product of Beijing Jing-Meng Stem Cell Technology Co., Ltd.), and is preferably the GMP S&XFM™-CD lymphocyte culture medium.

In the case that the lymphocyte culture medium used in step 1) is the AIM V® Medium CTS™, the activated culturing preferably is: adjusting the density of the cells to $1 \times 10^6$ to $3 \times 10^6$/mL using the AIM V® Medium CTS™ lymphocyte culture medium, adding zoledronic acid with the concentration ranging from $1 \mu g$/mL to $5 \mu g$/mL and recombinant human interleukin-2 with the concentration ranging from 500IU/mL to 1500 IU/mL, and culturing for 2 to 4 days in an environment with a temperature ranging from 36°C to 38°C and $CO_2$ saturated humidity of 5%; the activatedly culturing more preferably is: adjusting the density of the cells to $2 \times 10^6$/mL using the AIM V® Medium CTS™ lymphocyte culture medium, adding zoledronic acid with the concentration of $2 \mu g$/mL and recombinant human interleukin-2 with the concentration of 1000IU/mL, and culturing for 3 days in an environment with a temperature of 37°C and $CO_2$ saturated humidity of 5%.

In the case that the lymphocyte culture medium used in step 1) is the GMP S&XFM™-CD, the activatedly culturing preferably is one of the followings:

A: adjusting the density of the cells to 1x10$^6$ to 3×10$^6$/mL using the GMP S&XFM™-CD lymphocyte culture medium, adding zoledronic acid with the concentration ranging from 1μg/mL to 5μg/mL and recombinant human interleukin-2 with the concentration ranging from 500IU/mL to 1500 IU/mL, and culturing for 2 to 4 days in an environment with a temperature ranging from 36°C to 38°C and $CO_2$ saturated humidity of 5%; and the activatedly culturing more preferably is: adjusting the density of the cells to 2×10$^6$/mL using the GMP S&XFM™-CD lymphocyte culture medium, adding zoledronic acid with the concentration of 2μg/mL and recombinant human interleukin-2 with the concentration of 1000IU/mL, and culturing for 3 days in an environment with a temperature of 37°C and $CO_2$ saturated humidity of 5%;

B: adjusting the density of the cells to 1x10$^6$ to 3×10$^6$/mL using the GMP S&XFM™-CD lymphocyte culture medium, adding zoledronic acid with the concentration ranging from 1μg/mL to 5μg/mL, recombinant human interleukin-2 with the concentration ranging from 500IU/mL to 1500IU/mL, recombinant human interleukin-15 with the concentration ranging from Ing/mL to 100ng/mL (preferably from Ing/mL to 20ng/mL) and recombinant human interleukin-18 with the concentration ranging from Ing/mL to 100ng/mL (preferably from Ing/mL to 20ng/mL), and culturing for 2 to 4 days in an environment with a temperature ranging from 36°C to 38°C and $CO_2$ saturated humidity of 5%; the activatedly culturing more preferably is: adjusting the density of the cells to 2×10$^6$/mL using the GMP S&XFM™-CD lymphocyte culture medium, adding zoledronic acid with the concentration of 2μg/mL, recombinant human interleukin-2 with the concentration of 1000IU/mL, recombinant human interleukin-15 with the concentration of lOng/mL and recombinant human interleukin-18 with the concentration of lOng/mL, and culturing for 3 days in an environment with a temperature of 37°C and $CO_2$ saturated humidity of 5%;

C: adjusting the density of the cells to 1x10$^6$ to 3×10$^6$/mL using the GMP S&XFM™-CD lymphocyte culture medium, adding zoledronic acid with the concentration ranging from 1μg/mL to 5μg/mL, recombinant human interleukin-2 with the concentration ranging from 500IU/mL to 1500IU/mL, recombinant human interleukin-15 with the concentration ranging from Ing/mL to 100ng/mL (preferably from Ing/mL to 20ng/mL) and recombinant human interleukin-18 with the concentration ranging from Ing/mL to 100ng/mL (preferably from Ing/mL to 20ng/mL), and culturing for 0.5 to 1 day in an environment with a temperature ranging from 38.5°C to 39.5°C and $CO_2$ saturated humidity of 5%; the activatedly culturing more preferably is: adjusting the density of the cells to 2×10$^6$/mL using the GMP S&XFM™-CD lymphocyte culture medium, adding zoledronic acid with the concentration of 2μg/mL, recombinant human interleukin-2 with the concentration of 1000IU/mL, recombinant human interleukin-15 with the concentration of lOng/mL and recombinant human interleukin-18 with the concentration of lOng/mL, and culturing for 1 day in an environment with a temperature of 39°C and $CO_2$ saturated humidity of 5%.

The GMP S&XFM™-CD lymphocyte separation solution (the same name product of Beijing Jing-Meng Stem Cell Technology Co., Ltd., the same as sample density separation solution of medical device with record No.: Jinghai Machinery Equipment No. 20150002) is preferably used in proliferated culturing cord blood NK cells in step 2), and the operation preferably is: pipetting the cell fluid to a centrifuge tube, performing a centrifugation for 10 minutes with a centrifugal force of 150g, discarding the supernatant, adjusting the density of the cells to 0.5×10$^6$ to 2×10$^6$/mL (more preferably 1×10$^6$/mL) using the GMP S&XFM™-CD lymphocyte culture medium, adding recombinant human interleukin-2 with the concentration ranging from 500IU/mL to 1500IU/mL (more preferably 1000IU/mL), culturing in an environment with a temperature of 37°C and $CO_2$ saturated humidity of 5%, replenishing with fresh medium every other 2 to 3 days and adjusting the density of the cells to 0.5×10$^6$ to 2×10$^6$/mL (more preferably 1×10$^6$/mL), adding recombinant human interleukin-2 with the concentration ranging from 500IU/mL to 1500IU/mL (more preferably 1000IU/mL), and culturing for 18 to 24 days (more preferably 21 days), to obtain the cord blood NK cells.

A dedicated activation culture medium used in step 1) of the above ex vivo expansion method for the cord blood NK cells also belongs to the contents of the present disclosure. The dedicated activation culture medium is one of the following formulations:

an AIM V® Medium CTS™ lymphocyte culture medium added with zoledronic acid with the concentration ranging from 1μg/mL to 10μg/mL and recombinant human interleukin-2 with the concentration ranging from 200IU/mL to 2000IU/mL;

preferably, an AIM V® Medium CTS™ lymphocyte culture medium added with zoledronic acid with the concentration ranging from 1μg/mL to 5μg/mL and recombinant human interleukin-2 with the concentration ranging from 500IU/mL to 1500IU/mL; and

more preferably, an AIM V® Medium CTS™ lymphocyte culture medium added with zoledronic acid with the concentration of 2µg/mL and recombinant human interleukin-2 with the concentration of 1000IU/mL;

a GMP S&XFM™-CD lymphocyte culture medium added with zoledronic acid with the concentration ranging from 1µg/mL to 10µg/mL and recombinant human interleukin-2 with the concentration ranging from 200IU/mL to 2000IU/mL;

preferably, a GMP S&XFM™-CD lymphocyte culture medium added with zoledronic acid with the concentration ranging from 1µg/mL to 5µg/mL and recombinant human interleukin-2 with the concentration ranging from 500IU/mL to 1500IU/mL; and

more preferably, a GMP S&XFM™-CD lymphocyte culture medium added with zoledronic acid with the concentration of 2µg/mL and recombinant human interleukin-2 with the concentration of 1000IU/mL; or

a GMP S&XFM™-CD lymphocyte culture medium added with zoledronic acid with the concentration ranging from 1µg/mL to 10µg/mL, recombinant human interleukin-2 with the concentration ranging from 200IU/mL to 2000IU/mL, recombinant human interleukin-15 with the concentration ranging from Ing/mL to 100ng/mL and recombinant human interleukin-18 with the concentration ranging from 1ng/mL to 100ng/mL;

preferably, a GMP S&XFM™-CD lymphocyte culture medium added with zoledronic acid with the concentration ranging from 1µg/mL to 5µg/mL, recombinant human interleukin-2 with the concentration ranging from 500IU/mL to 1500IU/mL, recombinant human interleukin-15 with the concentration ranging from Ing/mL to 20ng/mL and recombinant human interleukin-18 with the concentration ranging from Ing/mL to 20ng/mL; and

more preferably, a GMP S&XFM™-CD lymphocyte culture medium added with zoledronic acid with the concentration of 2µg/mL, recombinant human interleukin-2 with the concentration of 1000IU/mL, recombinant human interleukin-15 with the concentration of IOng/mL and recombinant human interleukin-18 with the concentration of IOng/mL.

A dedicated proliferation culture medium used in step 2) of the above ex vivo expansion method for the cord blood NK cells also belongs to the content of the present disclosure. The dedicated proliferation culture medium is a GMP S&XFM™-CD lymphocyte culture medium added with recombinant human interleukin-2 with the concentration ranging from 200IU/mL to 2000IU/mL (preferably 1000IU/mL).

The cord blood NK cells obtained by the above ex vivo expansion method also belong to the content of the present disclosure, and the purity of the obtained cells exceeds 90%.

An application of the cord blood NK cells obtained by using the method according to the present invention in preparing tumor immunotherapy drugs or in a tumor immunotherapy also belongs to the content of the present disclosure.

Another object of the present disclosure is to provide an ex vivo expansion kit for cord blood NK cells.

The ex vivo expansion kit for the cord blood NK cells according to the present disclosure mainly includes the following reagents:

1): lymphocyte separation solution of the cord blood NK cells (the same name product of Beijing Jing-Meng Stem Cell Technology Co., Ltd.), or other commercially available lymphocyte separation solutions;

2): a cord blood NK cell activation culture medium, including: an AIM V® Medium CTS™ culture medium (Life Technology, USA) or a GMP S&XFM™-CD lymphocyte culture medium (product of Beijing Jing-Meng Stem Cell Technology Co., Ltd.), and zoledronic acid, recombinant human interleukin-2, recombinant human interleukin-15 and recombinant human interleukin-18; and

3): a cord blood NK cell proliferation culture medium, including: a GMP S&XFM™-CD lymphocyte culture medium (product of Beijing Jing-Meng Stem Cell Technology Co., Ltd.) and recombinant human interleukin-2.

The reagents in the ex vivo expansion kit for the cord blood NK cells is used based on the above ex vivo expansion method for the cord blood NK cells.

With the above solutions, a simply operable and safe ex vivo expansion method for the cord blood NK cells is provided according to the present invention. The present invention has the following advantageous effects as compared with existing methods for separating and expanding cord blood NK cells.

1): Cord blood mononuclear cells are directly used and no cell sorting step is required. That is, it is unnecessary to sort NK cells or hematopoietic stem cells, thereby simplifying a cell preparing process and greatly reducing preparing cost of the cord blood NK cells.

2): No trophoblast cells are required, and a safety risk brought by the trophoblast cells to the prepared cord blood NK cells is avoided.

3): No animal-source compositions are contained in the cell culture medium used for preparing the NK cells, hence the safety is good. In addition, the chemical compositions are clear so that the stability between batches is improved.

4): The yield of NK cells is high, the purity of the obtained cord blood NK cells is above 90%, and the total number of cells can reach $10^{10-11}$ per part of cord blood.

The ex vivo expansion method according to the present invention plays important roles in preparing tumor immunotherapy drugs and in tumor immunotherapy, and have broad application prospects.

BRIEF DESCRIPTION OF THE DRAWINGS

FIG. 1 shows an existing method for separating and expanding cord blood NK cells;

FIG. 2 shows a workflow of an ex vivo expansion method for cord blood NK cells according to the present invention;

FIG. 3 is a diagram showing cell morphologies of fresh cord blood NK cells and frozen resuscitation cord blood NK cells before and after an ex vivo expansion;

FIG. 4 is a diagram showing a growth curve of ex vivo expansion of fresh cord blood NK cells and a growth curve of ex vivo expansion of frozen resuscitation cord blood NK cells;

FIG. 5 shows results of the purities of the fresh cord blood NK cells and frozen resuscitation cord blood NK cells before and after ex vivo expansion, detected by a flow cytometer; and

FIG. 6 shows the tumor target cell killing effect of ex vivo expanded fresh cord blood NK cells and the tumor target cell killing effect of ex vivo expanded frozen resuscitation cord blood NK cells.

DETAILED DESCRIPTION OF THE EMBODIMENT(S) OF THE INVENTION

In view of the deficiency in separating and expanding cord blood NK cells in the prior art, the present invention aims to provide a method for ex vivo preparing cord blood NK cells.

An ex vivo expansion method for cord blood NK cells may include the following steps.

In step 1), the cord blood NK cells are activatedly cultured by: adjusting the density of cord blood mononuclear cells to $0.5\times10^6$ to $5\times10^6$/mL (preferably from $1\times10^6$ to $3\times10^6$/mL, and most preferably $2\times10^6$/mL) using a lymphocyte culture medium (described in detail later), inoculating cell suspension in a cell culture flask, adding zoledronic acid with the concentration ranging from $1\mu g$/mL to $10\mu g$/mL (preferably $1\mu g$/mL to $5\mu g$/mL, and most preferably $2\mu g$/mL) and recombinant human interleukin-2 (for forming a dedicated activation culture medium) with the concentration ranging from 200IU/mL to 2000 IU/mL (preferably 500IU/mL to 1500 IU/mL, and most preferably 1000IU/mL), and culturing for 1 to 5 days in an environment with a temperature ranging from 36°C to 40°C and $CO_2$ saturated humidity of 5%.

Specifically, the lymphocyte culture medium used in step 1) may be for example AIM V® Medium CTS™ (purchased from Life Technology, USA) or GMP S&XFM™-CD lymphocyte culture medium (the same name product of Beijing Jing-Meng Stem Cell Technology Co., Ltd., the same as the cell culture medium of medical device with record No.: Jinghai Machinery Equipment No. 20150008), and is preferably the GMP S&XFM™-CD lymphocyte culture medium. For detailed description of the GMP S&XFM™-CD lymphocyte culture medium, reference may also be made to Document 4 CN103146648A  with patent application No. 201310082166.8. The culture medium disclosed in document CN103146648A  essentially consists of IMDM (Iscove Modified Dulbecco Medium), L-glutamine, sodium bicarbonate, recombinant human insulin, human transferrin, human serum albumin, 2-mercaptoethanol, N-acetylcysteine, lipid, amino acid, vitamin, microelement, ferric citrate, hydrocortisone, cholamine and non-essential amino acid.

Different dedicated activation culture medium are correspondingly obtained according to the different types of lymphocyte culture medium as selected.

a) In the case that AIM V® Medium CTS™ lymphocyte culture medium is selected, the composition of the dedicated activation culture medium is: the AIM V® Medium CTS™ lymphocyte culture medium added with zoledronic acid with the concentration ranging from 1μg/mL to 10μg/mL (preferably from 1μg/mL to 5μg/mL, and most preferably 2μg/mL) and recombinant human interleukin-2 with the concentration ranging from 200IU/mL to 2000IU/mL (preferably from 500IU/mL to 1500IU/mL, and most preferably 1000IU/mL).

With the dedicated activation culture medium, the activatedly culturing method in step 1) preferably is: adjusting the density of cord blood mononuclear cells to $0.5\times10^6$ to $5\times10^6$/mL (preferably from $1\times10^6$ to $3\times10^6$/mL, and most preferably $2\times10^6$/mL) using the AIM V® Medium CTS™ lymphocyte culture medium, inoculating cell suspension in a cell culture flask, adding zoledronic acid with the concentration ranging from 1μg/mL to 10μg/mL (preferably from 1μg/mL to 5μg/mL, and most preferably 2μg/mL) and recombinant human interleukin-2 with the concentration ranging from 200IU/mL to 2000IU/mL (preferably from 500IU/mL to 1500IU/mL, and most preferably 1000IU/mL) into the AIM V® Medium CTS™ lymphocyte culture medium, and culturing in an environment with a temperature ranging from 36°C to 38°C (preferably 37°C) and $CO_2$ saturated humidity of 5% for 1 to 5 days (preferably 2 to 4 days, most preferably 3 days).

b) In the case that the GMP S&XFM™-CD lymphocyte culture medium is selected, the composition of the dedicated activation culture medium is: the GMP S&XFM™-CD lymphocyte culture medium added with zoledronic acid with the concentration ranging from 1μg/mL to 10μg/mL (preferably from 1μg/mL to 5μg/mL, and most preferably 2μg/mL) and recombinant human interleukin-2 with the concentration ranging from 200IU/mL to 2000IU/mL (preferably from 500IU/mL to 1500IU/mL, and most preferably 1000IU/mL).

With the dedicated activation culture medium, the activatedly culturing method in step 1) preferably is: adjusting the density of cord blood mononuclear cells to $0.5\times10^6$ to $5\times10^6$/mL (preferably from $1\times10^6$ to $3\times10^6$/mL, and most preferably $2\times10^6$/mL) using the GMP S&XFM™-CD lymphocyte culture medium, inoculating cell suspension in a cell culture flask, adding zoledronic acid with the concentration ranging from 1μg/mL to 10μg/mL (preferably from 1μg/mL to 5μg/mL, and most preferably 2μg/mL) and recombinant human interleukin-2 with the concentration ranging from 200IU/mL to 2000IU/mL (preferably from 500IU/mL to 1500IU/mL, and most preferably 1000IU/mL) into the GMP S&XFM™-CD lymphocyte culture medium, and culturing in an environment with a temperature ranging from 36°C to 38°C (preferably 37°C) and $CO_2$ saturated humidity of 5% for 1 to 5 days (preferably 2 to 4 days, most preferably 3 days).

c) In the case that the GMP S&XFM™-CD lymphocyte culture medium is selected, the composition of the dedicated activation culture medium is: the GMP S&XFM™-CD lymphocyte culture medium added with zoledronic acid with the concentration ranging from 1μg/mL to 10μg/mL (preferably from 1μg/mL to 5μg/mL, and most preferably 2μg/mL), recombinant human interleukin-2 with the concentration ranging from 200IU/mL to 2000IU/mL (preferably from 500IU/mL to 1500IU/mL, and most preferably 1000IU/mL), recombinant human interleukin-15 with the concentration ranging from Ing/mL to 100ng/mL (preferably from Ing/mL to 20ng/mL, and most preferably lOng/mL) and recombinant human interleukin-18 with the concentration ranging from Ing/mL to 100ng/mL (preferably from Ing/mL to 20ng/mL, and most preferably lOng/mL).

With the dedicated activation culture medium, the activatedly culturing method in step 1) preferably is: adjusting the density of cord blood mononuclear cells to $0.5\times10^6$ to $5\times10^6$/mL (preferably from $1\times10^6$ to $3\times10^6$/mL, and most preferably $2\times10^6$/mL) using the GMP S&XFM™-CD lymphocyte culture medium, inoculating cell suspension into a cell culture flask, adding zoledronic acid with the concentration ranging from 1μg/mL to 10μg/mL (preferably from 1μg/mL to 5μg/mL, and most preferably 2μg/mL), recombinant human interleukin-2 with the concentration ranging from 200IU/mL to 2000IU/mL (preferably from 500IU/mL to 1500IU/mL, and most preferably 1000IU/mL), recombinant human interleukin-15 with the concentration ranging from 1ng/mL to 100ng/mL (preferably from 1ng/mL to 20ng/mL, and most preferably lOng/mL) and recombinant human interleukin-18 with the concentration ranging from Ing/mL to 100ng/mL (preferably from Ing/mL to 20ng/mL, and most preferably lOng/mL) into the GMP S&XFM™-CD lymphocyte culture medium, and culturing in an environment with a temperature ranging from 36°C to 38°C (preferably 37°C) and $CO_2$ saturated humidity of 5% for 1 to 5 days (preferably 2 to 4 days, most preferably 3 days).

By using the dedicated activation culture medium and changing the culturing conditions, for example, culturing in an environment with a temperature ranging from 38°C to 40°C (preferably from 38.5°C to 39.5°C, and most preferably 39°C) and $CO_2$ saturated humidity of 5% for 0.5 to 3 days (preferably 0.5 to 1 day, most preferably 1 day), optimum conditions for activatedly culturing the cord blood NK cells in step 1) are obtained.

In step 2), the cord blood NK cells are proliferatedly cultured by: pipetting the cell fluid obtained in step 1) to a centrifuge tube, performing a centrifugation for 10 minutes with a centrifugal force of 150g, discarding the supernatant, adjusting the density of the cells to $0.5\times10^6$ to $5\times10^6$/mL (preferably from $0.5\times10^6$ to $2\times10^6$/mL, and most preferably $1\times10^6$/mL) using the GMP S&XFM™-CD lymphocyte culture medium, inoculating cell suspension into a cell culture flask, adding recombinant human interleukin-2 (for forming the dedicated proliferation culture medium) with the concentration ranging from 200IU/mL to 2000IU/mL (preferably from 500IU/mL to 1500IU/mL, and most preferably 1000IU/mL), culturing in an environment with a temperature ranging from 36°C to 38°C (preferably 37°C) and $CO_2$ saturated humidity of 5%, replenishing with fresh GMP S&XFM™-CD lymphocyte culture medium every other 2 to 3 days and adjusting the density of the cells to $0.5 \times 10^6$/mL to $5 \times 10^6$/mL (preferably from $0.5 \times 10^6$/mL to $2 \times 10^6$/mL, and most preferably $1 \times 10^6$/mL), adding recombinant human interleukin-2 with the concentration ranging from 200IU/mL to 2000IU/mL (preferably from 500IU/mL to 1500IU/mL, and most preferably 1000IU/mL), and culturing for 14 to 35 days (preferably 18 to 24 days, and most preferably 21 days), to obtain the cord blood NK cells.

The composition of the dedicated proliferation culture medium in the proliferatedly culturing in step 2) is: GMP S&XFM™-CD lymphocyte culture medium added with recombinant human interleukin-2 with the concentration ranging from 200IU/mL to 2000IU/mL (preferably from 500IU/mL to 1500 IU/mL, and most preferably 1000IU/mL).

The proliferatedly culturing of the cord blood NK cells in step 2) preferably is: pipetting the cell fluid to a centrifuge tube, centrifuging the cell fluid for 10 minutes with a centrifugal force of 150g (unit of the centrifugal force), discarding the supernatant, adjusting the density of the cells to $1 \times 10^6$/mL using the GMP S&XFM™-CD lymphocyte culture medium, inoculating cell suspension into a cell culture flask, adding recombinant human interleukin-2 with the concentration of 1000IU/mL into the cell culture flask, culturing in an environment with a temperature of 37°C and $CO_2$ saturated humidity of 5% for 21 days, during which fresh culture medium is replenished with every other 2 to 3 days and the density of cells is adjusted to $1 \times 10^6$/mL, to obtain the cord blood NK cells.

In the above method, the cord blood mononuclear cells used in step 1) are separated from the cord blood by the following steps 1.1 to 1.4.

In step 1.1, 100mL of fresh anticoagulation cord blood or frozen resuscitation cord blood is taken, and 100mL of PBS is added to dilute the cord blood.

In step 1.2, 20mL of lymphocyte separation solution (the same name product of Beijing Jing-Meng Stem Cell Technology Co., Ltd., the same as sample density separation solution of medical device with record No.: Jinghai Machinery Equipment No. 20150002; for details of the separation solution, reference may be made to Document 5 CN102533650A with patent No. ZL 201110456878.2,) is respectively added to ten centrifuge tubes each of a volume of 50mL. The 200mL of the diluted cord blood is slowly and uniformly added from above to the separation solution surface respectively, that is, 20mL of diluted cord blood is added to each of the centrifuge tubes to maintain a clear interface between the two liquids. Document CN102533650A discloses a compound lymphocyte separation solution comprising dextran with a molecular weight greater than 40 kD, pharmaceutically acceptable hydroxyethyl starch, and sodium diatrizoate or diatrizoate. The separation solution used in this step may be other commercially available lymphocyte separation solutions.

In step 1.3, centrifugation is performed for 20 to 40 minutes (preferably 30 minutes) at a room temperature and with a centrifugal force ranging from 400g to 1200g (preferably 980g, where g is a unit of centrifugal force. If other commercially available lymphocyte separation solutions are used in step 1.2, then an operation is executed according to the instructions of the other commercially available lymphocyte separation solutions).

In step 1.4, after the centrifugation, the solution in the centrifuge tube is divided into four layers with clear interfaces from top to bottom. The four layers are sequentially a light yellow plasma layer, a white membrane-like mononuclear cell layer, a transparent separation solution layer and a red erythrocyte layer from top to bottom. The white membrane-like mononuclear cell layer is carefully aspirated and put into another centrifuge tube, and is washed with PBS to obtain cord blood mononuclear cells.

Based on the above method, an ex vivo expansion kit for cord blood NK cells is further provided according to the present disclosure, which mainly includes the following reagents:

1): lymphocyte separation solution of the cord blood NK cells (the same name product of Beijing Jing-Meng Stem Cell Technology Co., Ltd., the same as sample density separation solution of medical device with record No.: Jinghai Machinery Equipment No. 20150002), or other commercially available lymphocyte separation solutions;

2): a cord blood NK cell activation culture medium, including: an AIM V® Medium CTS™ medium (purchased from Life Technology, USA) or a GMP S&XFM™-CD lymphocyte culture medium (the same name product of Beijing Jing-Meng Stem Cell Technology Co., Ltd., the same as cell culture medium of medical device with record No.: Jinghai Machinery Equipment No. 20150008), and other reagents described in the above steps 1) to 4), including zoledronic acid, recombinant human interleukin-2, recombinant human interleukin-15 and recombinant human interleukin-18; and

3): a cord blood NK cell proliferation culture medium, including: a GMP S&XFM™-CD lymphocyte culture medium (the same name product of Beijing Jing-Meng Stem Cell Technology Co., Ltd., the same as cell culture medium of medical device with record No.: Jinghai Machinery Equipment No. 20150008) and the above described reagent: recombinant human interleukin-2.

Hereinafter, the present invention is further described in conjunction with embodiments, and methods used in the embodiments are all conventional methods, unless otherwise stated.

Unless otherwise stated, the percentage concentration is a mass/mass (W/W, unit: g/100g) percentage concentration, mass/volume (W/V, unit: g/100mL) percentage concentration or volume/volume (V/V, unit: mL/100mL) percentage concentration.

The approaches for obtaining various biomaterials described in the embodiments are only intended to provide an experimental means for carrying out the present invention, and should not be considered as limiting the sources of the biomaterials of the present invention. In fact, the sources of the adopted biomaterials are extensive, and any biomaterial that can be obtained without violating laws and ethics can be used for replacement according to the instructions in the embodiments.

The embodiments are implemented according to the technical solutions of the present invention, and detailed embodiments and specific operation processes are given. The embodiments are helpful for understanding the present invention, but the scope of protection of the present invention is not limited to the following embodiments.

First embodiment: ex vivo expansion and detection of cord blood NK cells

I: ex vivo expansion of the cord blood NK cells (the first activatedly culturing scheme)

As shown in FIG. 1, the ex vivo expansion method for the cord blood NK cells according to the present invention includes the following steps.

In step 1), cord blood mononuclear cells are separated. Specifically:

In sub-step 1.1, 100mL of fresh anticoagulation cord blood (sample 1) and 100mL of frozen resuscitation cord blood (sample 2, the cord blood is provided by the Obstetrics and Gynecology Department of the Armed Police General Hospital, and approved by the hospital ethics committee) are taken, and 100mL of PBS (formulation: 8.0g of NaCl, 0.2g of KCl, 1.44g of $Na_2HPO_4$, and 0.24g of $KH_2PO_4$, adding distilled water to 1000mL, and adjusting the pH to 7.4) is added to dilute the cord blood.

In sub-step 1.2, 20mL of lymphocyte separation solution (the same name product of Beijing Jing-Meng Stem Cell Technology Co., Ltd., the same as sample density separation solution in medical device with record No.: Jinghai Machinery Equipment No. 20150002) is respectively added to ten centrifuge tubes each of a volume of 50mL. The diluted cord blood is slowly added from above to the separation solution surface respectively, that is, 20mL of diluted cord blood is added to each of the centrifuge tubes to maintain a clear interface between the two liquids.

In sub-step 1.3, centrifugation is performed for 30 minutes at a room temperature and with a centrifugal force of 980g.

In sub-step 1.4, after the centrifugation, the solution in the centrifuge tube is divided into four layers with clear interfaces from top to bottom. The four layers are sequentially a light yellow plasma layer, a white membrane-like mononuclear cell layer, a transparent separation solution layer and a red erythrocyte layer from top to bottom. The white membrane-like mononuclear cell layer is carefully aspirated and put into another centrifuge tube, and is washed with PBS for 2 to 3 times, to obtain mononuclear cells.

In step 2), the cord blood NK cells are activatedly cultured.

The density of mononuclear cells is adjusted to $2\times10^6$/mL (or any density ranging from $0.5\times10^6$/mL to $5\times10^6$/mL, and preferably ranging from $1\times10^6$/mL to $3\times10^6$/mL, wherein being less than $0.5\times10^6$/mL or greater than $5\times10^6$/mL is not advantageous for the activation of the NK cells) using the AIM V® Medium CTS™ lymphocyte culture medium (purchased from Life Technology Company, USA). Mononuclear cell suspension is inoculated into a cell culture flask. Then, zoledronic acid (Zetai, Novartis Pharmaceuticals) with the concentration of 2μg/mL (or any concentration ranging from 1μg/mL to 10μg/mL, and preferably ranging from 1μg/mL to 5μg/mL; the activation of the NK cells is affected if the concentration is less than 1μg/mL and is only slightly affected if the concentration is greater than 10μg/mL) and recombinant human interleukin-2 (De Lusheng, Beijing SiHuanShengWu) with the concentration of 1000IU/mL (or any concentration ranging from 200IU/mL to 2000IU/mL, and preferably ranging from 500IU/mL to 1500IU/mL; the activation of the NK cells is affected if the concentration is less than 200IU/mL, and the cells are prone to death if the concentration is greater than 2000IU/mL) are added into the AIM V® Medium CTS™ lymphocyte culture medium. Culturing is performed for 3 days (or any time duration ranging from 1 day to 5 days, preferably ranging from 2 days to 4 days; the activation of the NK cells is low if the culturing is performed for less than 1 day, and the activation of the NK cells is only slightly affected if the culturing is performed for more than 5 days) in an environment with a temperature of 37°C (or any temperature ranging from 36°C to 38°C; the cells can not normally grow if the temperature is lower than 36°C or higher than 38°C) and $CO_2$ saturated humidity of 5%.

In step 3), the cord blood NK cells are proliferatedly cultured.

The cell fluid is pipetted to a centrifuge tube. Centrifugation is performed for 10 minutes with a centrifugal force of 150g. The supernatant is discarded. The density of the cells is adjusted to $1\times10^6$/mL (or any density ranging from $0.5\times10^6$ to $5\times10^6$/mL, preferably from $0.5\times10^6$ to $2\times10^6$/mL, wherein being less than $0.5\times10^6$/mL or greater than

$5\times10^6$/mL is not advantageous for the proliferation of the NK cells) using the GMP S&XFM™-CD lymphocyte culture medium (the same name product of Beijing Jing-Meng Stem Cell Technology Co., Ltd., the same as cell culture medium of medical device with record No.: Jinghai Machinery Equipment No. 20150008). The cell fluid is inoculated to a cell culture flask. Recombinant human interleukin-2 with the concentration of 1000IU/mL (or any concentration ranging from 200IU/mL to 2000IU/mL, preferably ranging from 500IU/mL to 1500IU/mL; the proliferation of the NK cells is affected if the concentration is less than 200IU/mL, and the cells are prone to death if the concentration is greater than 2000IU/mL) is added. Culturing is performed in an environment with a temperature of 37°C (or any temperature ranging from 36°C to 38°C; the cells can not normally grow if the temperature is lower than 36°C or higher than 38°C) and $CO_2$ saturated humidity of 5%. Fresh lymphocyte culture medium is replenished with every other 2 to 3 days to adjust the density of the cells to a value ranging from $0.5\times10^6$/mL to $5\times10^6$/mL (more preferably $1\times10^6$/mL, and preferably ranging from $0.5\times10^6$/mL to $2\times10^6$/mL; the proliferation of the cells is slow if the density of the cells is less than $0.5\times10^6$/mL, and a contact inhibition is easy to form quickly for the cells, which affects the proliferation of the cells, if the density of the cells is greater than $5\times10^6$/mL). Recombinant human interleukin-2 with the concentration of 1000IU/mL (or any concentration ranging from 200IU/mL to 2000 IU/mL, and preferably from 500IU/mL to 1500IU/mL) is added. Culturing is performed for 14 to 35 days (preferably 18 to 24 days, and most preferably 21 days) to obtain the cord blood NK cells. At the 21th day, the number of the expanded cells is large and the killing activity of the cells is the greatest. Therefore, after the 21th day, the cells enters a steady phase and the killing activity of the cells is decreased. Therefore, the 21th day is preferably taken as a harvesting point for the cord blood NK cells.

II: detection of the ex vivo expanded cord blood NK cells

1: counting the cord blood NK cells, including:

1) gently blowing the cord blood NK cell suspension, to form NK mononuclear cell suspension;

2) taking 20μl of the NK mononuclear cell suspension, adding the NK mononuclear cell suspension into 20μl of 0.2% trypan blue staining solution (purchased from SIGMA Company), suctioning repeatedly and mixing gently so that the mixture is uniform;

3) taking 20μl of the mixture and adding the taken mixture into a counting plate groove of a Countstar (IC1000) cell counter (purchased from Shanghai Ruiyu Biotechnology Co., Ltd.); and

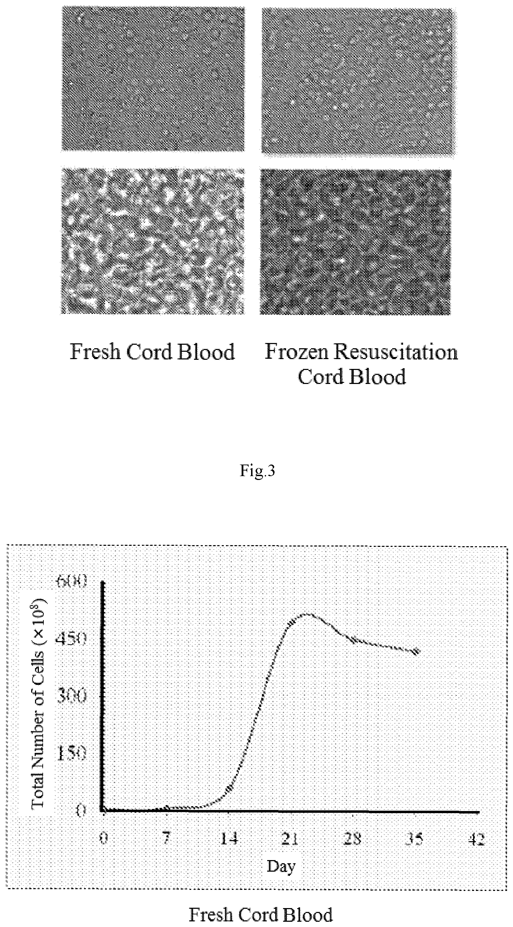

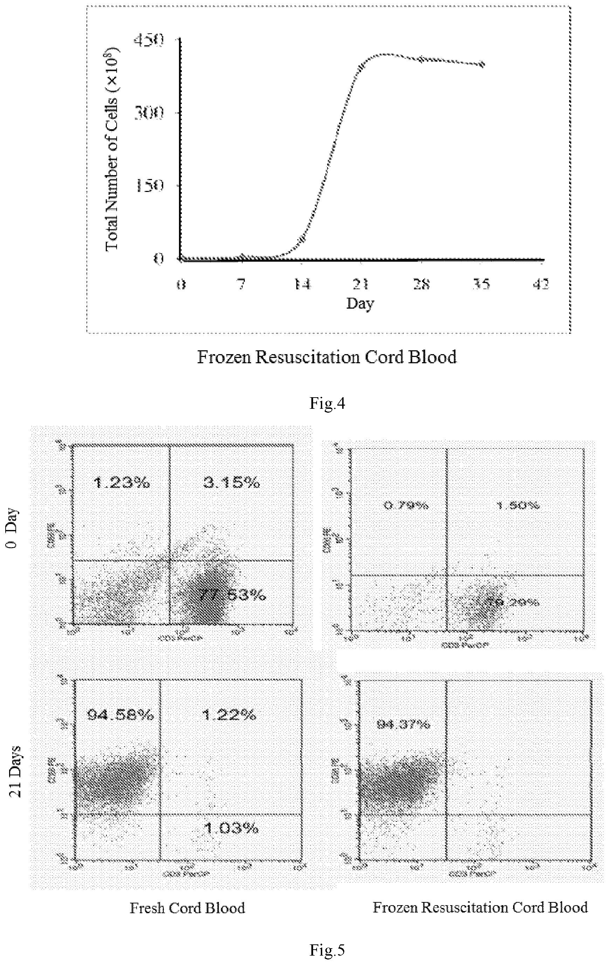

4) standing still for 1 minute and reading a measurement.
Results: after the ex vivo activating and expansion culturing of the cord blood NK cells separated from the fresh anticoagulation cord blood or frozen resuscitation cord blood (obtained by the above method in I), by observing with a microscope, the cell morphology is gradually changed from the circle shape of different sizes before the expansion to uniform and irregular cell morphology, and the volume of the cell body and the volume of the cell nucleus are increased (the cells expanded and cultured for 21 days are shown in FIG. 3). The number of the cells is proliferated from the original $4.36\times10^8$ /mL (fresh cord blood) and $3.25\times10^8$/mL (frozen resuscitation cord blood) to $5.08\times10^{10}$/mL (fresh cord blood) and $3.94\times10^{10}$/mL (frozen resuscitation cord blood) after 21 days of expansion and culturing, as shown in FIG. 4. It can also be seen from FIG. 4 that the period from the 14th to the 21th expanding and culturing day is a rapid proliferation phase for cells, and the period from 21th day to the 35th day is a slow declining phase. Therefore, the cord blood NK cells can be harvested in the period from the 14th day to the 35th days as required.

2: detecting the purity of the cord blood NK cells using a flow cytometer, including:

1) taking mononuclear cell suspension and centrifuging for 10 minutes with a centrifugal force of 150g;

2) resuspending the cell precipitation with PBS, adjusting the concentration of the cells to $1\times10^6$/100μl, and placing the solution into a flow-type detection tube;

3) adding antibodies CD56 and CD3 (purchased from BD company), gently blowing and uniformly mixing the solution, incubating for 30 minutes at 4°C in the dark, and meanwhile setting an isotype control;

4) centrifuging at 1500g for 5 minutes and discarding the supernatant; and

5) adding 100μl of PBS, gently blowing and uniformly mixing, and putting the solution on a machine for detection. Results: the purity of the NK cells expanded and cultured for 21 days in I is increased from the original 1.23% (fresh cord blood) and 0.79% (frozen resuscitation cord blood) to 94.58% (fresh cord blood) and 94.37% (frozen resuscitation cord blood) respectively, as shown in FIG. 5.

3: measuring a tumoricidal activity of the cord blood NK cells with a CCK-8 method, including:

1) taking well-grown A549 cells (human lung adenocarcinoma cells, derived from ATCC) as target cells, and digesting with 0.25% trypsin;

2) performing a trypan blue staining counting, and adjusting the concentration of the cells to $5 \times 10^4$/ mL;

3) adding 100ul per well in a 96-well plate and placing the plate in a 37°C and 5% $CO_2$ environment for incubating overnight;

4) taking cord blood NK cell suspension as effector cells, and adjusting the concentration of the cells to $2 \times 10^6$/mL;

5) adding into the 96-well plate based on an effective target ratio of 5:1, 10:1 and 20:1 in triplicate for each group;

6) culturing for 4h in an incubator with a temperature of 37°C and 5% $CO_2$;

7) adding 15μl of CCK-8 (purchased from Biyuntian) to each well and continuing incubating for 2h;

8) detecting an OD value of a wavelength of 450nm with a microplate reader; and

9) calculating a kill rate:

$$\text{Kill rate (\%)} = [1- (\text{OD value of experimental group - OD value of individual effector cells})$$
$$\div \text{OD value of individual target cells}] \times 100\%.$$

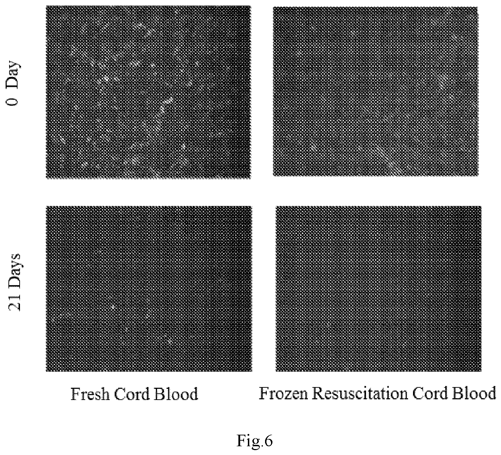

Results: it is observed under the fluorescence microscope that, the killing ability of the cord blood NK cells after 21 days of ex vivo expansion and culturing in I to A549 tumor cells is improved greatly, as shown in FIG. 6.

The above detection results show that the cord blood NK cells expanded ex vivo according to the method of the present invention has a large quantity, high purity, and a high killing activity to tumor cells.

Second embodiment: ex vivo expansion of cord blood NK cells (the second activatedly culturing scheme)

As shown in FIG. 1, the ex vivo expansion of the cord blood NK cells includes the following steps.

In step 1), cord blood mononuclear cells are separated from frozen resuscitation cord blood.

10ml of frozen resuscitation cord blood (sample 3) is taken by using the same method as the first embodiment.

In step 2), the cord blood NK cells are activatedly cultured (a control group is the first embodiment).

The density of the mononuclear cells is adjusted to $2 \times 10^6$/mL (or any density ranging from $0.5 \times 10^6$/mL to $5 \times 10^6$/mL, and preferably from $1 \times 10^6$/mL to $3 \times 10^6$/mL) using the GMP S&XFM™-CD lymphocyte culture medium (the same name product of Beijing Jing-Meng Stem Cell Technology Co., Ltd., the same as the cell culture medium of medical device with record No.: Jinghai Machinery Equipment 20150008). Cell suspension is inoculated into a cell culture flask, and then zoledronic acid (Zetai, Novartis Pharmaceuticals) with the concentration of 2μg/mL (or any concentration ranging from 1μg/mL to 10μg/mL, and preferably ranging from 1μg/mL to 5μg/mL) and recombinant human interleukin-2 (De Lusheng, Beijing SiHuanShengWu) with the concentration of 1000IU/mL (or any concentration ranging from 200IU/mL to 2000IU/mL, and preferably ranging from 500IU/mL to 1500IU/mL) are added into the GMP S&XFM™-CD lymphocyte culture medium. Culturing is performed for 1 to 5 days (preferably 2 to 4 days, and most preferably 3 days; the activation of the NK cells is low if the culturing is performed for less than 1 day, and the activation of the NK cells is only slightly affected if the culturing is performed for more than 5 days) in an environment with a temperature of 37°C (or any temperature ranging from 36°C to 38°C) and $CO_2$ saturated humidity of 5%.

The effect that the same added reagent has on the cell activation is the same as that described in the first embodiment, and is not repeatedly described hereinafter.

In step 3), the cord blood NK cells are proliferatedly cultured.

This step is the same as that in the first embodiment.

Third embodiment: ex vivo expansion and detection of cord blood NK cells (the third activatedly culturing scheme).

As shown in FIG. 1, the ex vivo expansion of the cord blood NK cells includes the following steps.

In step 1), cord blood mononuclear cells are separated from frozen resuscitation cord blood.

10ml of frozen resuscitation cord blood (sample 3) is taken using the same method as the first embodiment.

In step 2), the cord blood NK cells are activatedly cultured.

The density of the mononuclear cells is adjusted to $2\times10^6$/mL (or any density ranging from $0.5\times10^6$ to $5\times10^6$/mL, and preferably from $1\times10^6$ to $3\times10^6$/mL) using the GMP S&XFM™-CD lymphocyte culture medium (the same name product of Beijing Jing-Meng Stem Cell Technology Co., Ltd., the same as the cell culture medium of medical device with record No.: Jinghai Machinery Equipment 20150008). Cell suspension is inoculated into a cell culture flask. Then, zoledronic acid (Zetai, Novartis Pharmaceuticals) with the concentration of 2μg/mL (or any concentration ranging from 1μg/mL to 10μg/mL, and preferably ranging from 1μg/mL to 5μg/mL), recombinant human interleukin-2 (De Lusheng, Beijing SiHuanShengWu) with the concentration of 1000IU/mL (or any concentration ranging from 200IU/mL to 2000IU/mL, and preferably ranging from 500IU/mL to 1500IU/mL), recombinant human interleukin-15 (purchased from Peprotech Company) with the concentration of lOng/mL (or any concentration ranging from Ing/mL to 100ng/mL, and preferably from Ing/mL to 20ng/mL; there is no effect on the activation of the NK cells if the concentration is less than 1ng/mL, and the activation of the NK cells is only slightly affected if the concentration is greater than 100ng/mL) and recombinant human interleukin-18 (purchased from Peprotech Company) with the concentration of lOng/mL (or any concentration ranging from Ing/mL to 100ng/mL, and preferably from Ing/mL to 20ng/mL; there is no effect on the activation of the NK cells if the concentration is less than 1ng/mL, and the activation of the NK cells is only slightly affected if the concentration is greater than 100ng/mL) are added into the GMP S&XFM™-CD lymphocyte culture medium. Culturing is performed for 3 days (or any time duration ranging from 1 to 5 days, preferably from 2 to 4 days; the activation of the NK cells is low if the culturing is performed for less than 1 day, and the activation of the NK cells is only slightly affected if the culturing is performed for more than 5 days) in an environment with a temperature of 37°C (or any temperature ranging from 36°C to 38°C) and $CO_2$ saturated humidity of 5%.

The effect that the same added reagent has on the cell activation is the same as that described in the first embodiment, and is not repeatedly described hereinafter.

In step 3), the cord blood NK cells are proliferatedly cultured.

This step is the same as that in the first embodiment.

Fourth embodiment: ex vivo expansion of cord blood NK cells (the fourth activatedly culturing scheme).

As shown in FIG. 1, the ex vivo expansion of the cord blood NK cells includes the following steps.

In step 1), cord blood mononuclear cells are separated from frozen resuscitation cord blood.

10ml of frozen resuscitation cord blood (sample 3) is taken using the same method as the first embodiment.

In step 2), the cord blood NK cells are activatedly cultured.

The activation culture medium which is same as that used in the third activatedly culturing scheme is used, and the culturing condition is changed to: culturing for 1 day (or any time duration ranging from 0.5 to 3 days; the activation of the NK cells is low if the culturing is performed for less than 0.5 day, and the NK cells may be prone to death if the culturing is performed for more than 3 days) in an environment with a temperature of 39°C±0.5°C (or any temperature ranging from 38°C to 40°C; with the increased temperature, on one hand, it is advantageous for accelerating the activation of the cord blood NK cells and improving the activation efficiency, and on the other hand, the expansion amplification, purity and biological activity of the cord blood NK cells may be improved; however, if the temperature is higher than 40°C, the NK cells cannot adapt and may die) and $CO_2$ saturated humidity of 5%.

In step 3), the cord blood NK cells are proliferatedly cultured.

This step is the same as that in the first embodiment.

A detection is performed on the cord cell NK cells obtained in the methods according to the above embodiments.

Cord blood NK cell detection

The cord blood NK cells obtained by ex vivo expanding 10ml of frozen resuscitation cord blood (sample 3) is detected using the method described in part II of the first embodiment (a parallel operation is performed by taking sample 3 as a starting blood source and using the methods according to the first to fourth embodiments).

1: counting the cord blood NK cells

After the cord blood mononuclear cells separated from the frozen resuscitation cord blood are expanded and cultured for 21 days ex vivo, the cell expansion results are shown in Table 1-1 and Table 1-2.

In the present invention, NK cells with a high purity can be obtained by the two steps of activating and expanding the cord blood mononuclear cells (here, the proportion of NK cells in the mononuclear cells is low, see Table 2, and the proportion of 0-day NK cells is only 9.56%). Table 1-1 shows the total numbers of cells (including expanded NK cells and other cells) counted before and after the expansion. The absolute number of the NK cells is a value obtained by multiplying the total number of the cells and the proportion of the NK cells, that is, data of Table1-1×Table 2 = Table 1-2.

The 0-day cord blood mononuclear cells are cells before activation and expansion according to the present invention (including a small quantity of NK cells and other cells), and can be taken as a control; the 0-day cord blood NK cells refer to NK cells therein. The 21-day cord blood NK cell are the total number of cells after the mononuclear cells are expanded for 21 days. In this way, since the purity of the NK cells is high and most of the cells are NK cells, the cells obtained by the 21 days of expansion are called NK cells (precisely, mononuclear cells mainly composed of NK cells).

It can be seen from the results that, through all of the first embodiment to the fourth embodiment, the total number of the cord blood cells is expanded by a hundredfold (Table 1-1), the absolute number of the NK cells is expanded by a thousandfold (Table 1-2), and a comparison of the expansion effects is: the fourth embodiment > the third embodiment > the second embodiment > the first embodiment.

Table 1-1. Total number of cord blood cells and expansion amplification (10ml of cord blood)

| | First embodiment | Second embodiment | Third embodiment | Fourth embodiment |
|---|---|---|---|---|
| Total number of 0-day cord blood mononuclear cells ($\times 10^7$) | 3.74 | 3.74 | 3.74 | 3.74 |
| Total number of 21-day expansion cells ($\times 10^7$) | 425 | 536 | 659 | 782 |
| Expansion amplification of the total number of cells | 113.64 | 143.32 | 176.20 | 209.09 |

Table1-2. Absolute number of cord blood NK cells and expansion amplification (10ml of cord blood)

| | First embodiment | Second embodiment | Third embodiment | Fourth embodiment |
|---|---|---|---|---|
| Absolute number of 0-day cord blood NK cells ($\times 10^6$) | 3.58 | 3.58 | 3.58 | 3.58 |
| Absolute number of 21-day cord blood NK cells ($\times 10^6$) | 3854.3 | 4955.9 | 6178.1 | 7459.5 |
| Expansion amplification of cord blood NK cells | 1078.00 | 1386.08 | 1727.93 | 2086.32 |

2: detecting the purity of the cord blood NK cells by using a flow cytometer.

NK cell purity results obtained by activating and expanding the cord blood mononuclear cells ex vivo for 21 days are shown in Table 2, where the cord blood mononuclear cells are separated from the frozen resuscitation cord blood.

Table 2. Purities of cord blood NK cells

| | First embodiment | Second embodiment | Third embodiment | Fourth embodiment |
|---|---|---|---|---|
| Purity of 0-day cord blood NK cells | 9.56% | 9.56% | 9.56% | 9.56% |
| Purity of 21-day cord blood NK cells | 90.69% | 92.46% | 93.75% | 95.39% |

The data in Table 2 indicates that, after the expansions in the above embodiments, the proportions of NK cells in the cord blood mononuclear cells are all improved from the original 9.56% to the purities of the NK cells greater than or equal to 90%. The purities obtained by the first embodiment to the fourth embodiment have the following relationship: fourth embodiment > third embodiment > second embodiment > first embodiment.

3: measuring a tumoricidal activity of the cord blood NK cells with a CCK-8 method.

The experiment takes 0-day cord blood mononuclear cells (cells before activation and expansion) as a negative control, and takes CIK cells (an immune cell that kills tumors) as a positive control. For the culturing of the CIK cells, reference may be made to a Reference Document (Document 6: Adoptive immunotherapy with cytokine-induced killer cells generated with a new good manufacturing practice-grade protocol. Cytotherapy, 2012; Early Online: 1-10. DOI: 10.3109/14653249.2012.681038).

The results are shown in Table 3. By observing under a fluorescence microscope, it is shown that the cord blood NK cells harvested after 21 days of ex vivo expansion according to the embodiments all have a strong killing ability to A549 tumor cells, and as compared with the positive control CIK cells, the NK cells obtained after the expansion according to the present invention are more active. The killing abilities of the four embodiments have the following relationship under the same effective target ratio: fourth embodiment > third embodiment > second embodiment > first embodiment. Therefore, the cells expanded according to the fourth embodiment have the strongest cell killing ability.

Table 3. Killing ability of cord blood NK cells to A549 tumor cells

|  | 0 day | First embodiment (21 days) | Second embodiment (21 days) | Third embodiment (21 days) | Fourth embodiment (21 days) | CIK cell |
|---|---|---|---|---|---|---|
| Effective target ratio (5:1) | 9.71% | 52.18% | 54.21% | 77.52% | 79.11% | 30.56% |
| Effective target ratio (10:1) | 17.42% | 60.77% | 63.85% | 85.63% | 87.27% | 42.78% |
| Effective target ratio (20:1) | 23.74% | 72.90% | 71.53% | 88.91% | 91.45% | 55.61% |

It can be seen from the above embodiments that, the ex vivo expansion method for the cord blood NK cells according to the present invention has a simple operation and is safe, and comparison results between the method according to the present invention and the existing methods for separating and expanding the cord blood NK cells (the prior art) are shown in Table 4.

Table 4 Comparison between the conventional technologies and technologies according to the present invention

|  | Technology | | | Related products | | | Cellular properties | |
|---|---|---|---|---|---|---|---|---|
|  | Cost | Versatility | Safety | Sorting reagent | Medium and its additives | trophoblast layer | Purity | Yield (perpart of cord blood) |
| Celgene | High | Poor | Poor | NK negative selection kit | IMDM, fetal bovine serum, IL-2, PHA-P | K562 pretreated with mitomycin C | >80% | $10^{9-10}$ |
| Glycostem | High | Poor | Not bad | CD34 positive selection kit | GBGM, 10% human serum, 12 kinds of cytokines | No need | >90% | $10^{9-10}$ |
| The present invention | Low | Good | Good | No need | Lymphocyte culture medium, IL-2, Zetai | No need | >90% | $10^{10-11}$ |
| Notes: the amount of one part of cord blood is 100ml (typically, the amount of one part of cord blood is in a range from 50ml to 120ml). | | | | | | | | |

Fifth embodiment: ex vivo expansion kit for cord cell NK cells.

The ex vivo expansion kit for the cord blood NK cells according to the present disclosure mainly includes the following reagents:

1): lymphocyte separation solution of the cord blood NK cells (the same name product of Beijing Jing-Meng Stem Cell Technology Co., Ltd., the same as sample density separation solution of medical device with record No.: Jinghai Machinery Equipment No. 20150002), or other commercially available lymphocyte separation solution;

2): a cord blood NK cell activation culture medium: an AIM V® Medium CTS™ medium (purchased from Life Technology, USA) or a GMP S&XFM™-CD lymphocyte culture medium (the same name product of Beijing Jing-Meng Stem Cell Technology Co., Ltd., the same as cell culture medium of medical device with record No.: Jinghai Machinery Equipment No. 20150008), and zoledronic acid, recombinant human interleukin-2, recombinant human interleukin-15 and recombinant human interleukin-18; and

3): a cord blood NK cell proliferation culture medium: a GMP S&XFM$^{TM}$-CD lymphocyte culture medium (the same name product of Beijing Jing-Meng Stem Cell Technology Co., Ltd., the same as cell culture medium of medical device with record No.: Jinghai Machinery Equipment No. 20150008) and recombinant human interleukin-2.

The kit may be used by making reference to the methods according to the first to fourth embodiments.
In summary, the present invention has the following advantages.

1): no cell sorting is required.

2): no trophoblast cell is required.

3): operations are simple and a good versatility is achieved.

4): the cost is low.

5): the adopted reagents contain no animal-source compositions, hence a good safety is achieved.

6): the yield of NK cells is high. The purity of the obtained cord blood NK cells may be above 90%, and the total number of the cells can reach $10^{10\text{-}11}$ cells per part of cord blood (the sample is 100ml according to the first embodiment).

7): the killing ability to tumor cells is high.

Industrial Applicability

In the present invention, the cord blood NK cells are obtained by activatedly culturing and proliferatedly culturing separated cord blood mononuclear cells. It is not required to sort cells, and no trophoblast cells are required. The technical solutions according to the present invention have simple operations, a good versatility and a low cost. The technical solutions according to the present invention also have a good safety since the reagent contains no animal-source compositions. In addition, the yield of harvested NK cells is high, the total number of cells can reach $10^{10\text{-}11}$ cells per part of cord blood, the purity is above 90%, and the killing ability to tumor cells is high. The present invention can be applied to the preparation of tumor immunotherapy drugs.

**Claims**

1.  An ex vivo expansion method for cord blood Natural killer (NK) cells, **characterized in that** the ex vivo expansion method comprises the following steps:

    step 1): activatedly culturing the cord blood NK cells by: adjusting the density of cord blood mononuclear cells to $0.5\times10^6$ to $5\times10^6$/mL using a lymphocyte culture medium, adding at least zoledronic acid with the concentration ranging from $1\mu g$/mL to $10\mu g$/mL and recombinant human interleukin-2 with the concentration ranging from 200IU/mL to 2000IU/mL, culturing for 1 to 5 days in an environment with a temperature ranging from 36°C to 40°C and $CO_2$ saturated humidity of 5%, and collecting cell fluid; and

step 2): proliferately culturing the cord blood NK cells by: obtaining cells from the cell fluid collected in step 1), adjusting the density of the cells to $0.5\times10^6$ to $5\times10^6$/mL using a lymphocyte culture medium, adding recombinant human interleukin-2 with the concentration ranging from 200IU/mL to 2000IU/mL, culturing in an environment with a temperature ranging from 36°C to 38°C and $CO_2$ saturated humidity of 5%, replenishing with fresh culture medium every other 2 to 3 days and adjusting the density of the cells to $0.5\times10^6$ to $5\times10^6$/mL, and culturing for 14 to 35 days to harvest the cord blood NK cells.

**2.** The ex vivo expansion method according to claim 1, **characterized in that** the cord blood mononuclear cells used in step 1) are separated from fresh anticoagulation cord blood or frozen resuscitation cord blood by:

1.1: taking the fresh anticoagulation cord blood or frozen resuscitation cord blood, and diluting it with Phosphate Buffered Saline (PBS) of a volume which is 1 to 2 times the volume of the cord blood;

1.2: adding slowly the diluted cord blood from above to a lymphocyte separation solution, and maintaining a clear interface between the diluted cord blood and the lymphocyte separation solution, wherein the volume of the diluted cord blood is equal to the volume of the lymphocyte separation solution;

1.3: centrifuging for 20 to 30 minutes at a room temperature and with a centrifugal force of 980g; and

1.4: after the centrifugation, aspirating a white membrane-like mononuclear cell layer, which is a second layer from the top, and washing the mononuclear cell layer with the PBS, to obtain cord blood mononuclear cells.

## Patentansprüche

**1.** Ex-vivo-Expansionsverfahren für natürliche Killer (NK)-Zellen aus Nabelschnurblut, **dadurch gekennzeichnet, dass** das Ex-vivo-Expansionsverfahren folgende Schritte umfasst:

Schritt 1): aktiviertes Kultivieren der Nabelschnurblut-NK-Zellen durch: Einstellen der Dichte von mononuklearen Nabelschnurblutzellen auf $0,5\times10^6$ bis $5\times10^6$/ml mithilfe eines Lymphocytenkulturmediums, Zusetzen von wenigstens Zoledronsäure mit einer Konzentration von 1 $\mu$g/ml bis 10 $\mu$g/ml und rekombinantem humanem Interleukin-2 mit einer Konzentration von 200 IU/ml bis 2000 IU/ml, Kultivieren für 1 bis 5 Tage in einer Umgebung mit einer Temperatur von 36°C bis 40°C und einer $CO_2$-gesättigten Feuchtigkeit von 5%, und Auffangen von Zellfluid; und

Schritt 2): proliferierendes Kultivieren der Nabelschnurblut-NK-Zellen durch: Erlangen von Zellen aus dem in Schritt 1) aufgefangenen Zellfluid, Einstellen der Dichte der Zellen auf $0,5\times10^6$ bis $5\times10^6$/ml mithilfe eines Lymphocytenkulturmediums, Zusetzen von rekombinantem humanem Interleukin-2 mit einer Konzentration von 200 IU/ml bis 2000 IU/ml, Kultivieren in einer Umgebung mit einer Temperatur von 36°C bis 38°C und einer $CO_2$-gesättigten Feuchtigkeit von 5%, Auffüllen mit frischem Kulturmedium alle 2 bis 3 Tage und Einstellen der Dichte der Zellen auf $0,5\times10^6$ bis $5\times10^6$/ml, und Kultivieren für 14 bis 35 Tage zum Gewinnen der Nabelschnurblut-NK-Zellen.

**2.** Ex-vivo-Expansionsverfahren nach Anspruch 1, **dadurch gekennzeichnet, dass** die in Schritt 1) verwendeten mononuklearen Nabelschnurblutzellen aus frischem Antikoagulationsnabelschnurblut oder gefrorenem wiederbelebtem Nabelschnurblut abgetrennt werden durch:

1.1: Verdünnen des frischen Antikoagulationsnabelschnurbluts oder des gefrorenen wiederbelebten Nabelschnurbluts mit phosphatgepufferter Kochsalzlösung (PBS) mit einem 1- bis 2-fachen Volumen des Volumens des Nabelschnurbluts;

1.2: langsames Zusetzen des verdünnten Nabelschnurbluts oben zu einer Lymphocytentrennlösung und beibehalten einer klaren Grenzfläche zwischen dem verdünnten Nabelschnurblut und der Lymphocytentrennlösung, wobei das Volumen des verdünnten Nabelschnurbluts gleich dem Volumen der Lymphocytentrennlösung ist;

1.3: Zentrifugieren für 20 bis 30 Minuten bei Raumtemperatur und mit einer Zentrifugalkraft von 980 g; und

1.4: nach der Zentrifugation, Ansaugen einer weißen membranartigen Schicht der mononuklearen Zelle, die eine zweite Schicht von oben ist, und Waschen der Schicht der mononuklearen Zelle mit der PBS, um mononukleare Nabelschnurblutzellen zu erlangen.

**Revendications**

1.  Un procédé d'expansion ex vivo destiné aux cellules tueuses naturelles (NK) du sang ombilical, **caractérisé en ce que** le procédé d'expansion ex vivo comprend les étapes suivantes :

    étape 1) : culture activée des cellules tueuses naturelles du sang ombilical par : ajustement de la densité de cellules mononucléaires du sang ombilical de $0,5 \times 10^6$ à $5 \times 10^6$/mL à l'aide d'un milieu de culture lymphocytaire, ajout d'une concentration d'acide zolédronique au moins comprise entre 1 $\mu$g/mL et 10 $\mu$g/mL et d'une concentration d'interleukine-2 humaine recombinante comprise entre 200 IU/mL et 2000 IU/mL, culture durant 1 à 5 jours dans un environnement dont la température est comprise entre 36°C et 40°C et à humidité saturée de $CO_2$ de 5%, et collecte du liquide cellulaire; et

    étape 2): culture proliférative des cellules tueuses naturelles du sang ombilical par: obtention des cellules du liquide cellulaire collecté à l'étape 1), ajustement de la densité cellulaire de $0,5 \times 10^6$ à $5 \times 10^6$/mL à l'aide d'un milieu de culture lymphocytaire, ajout d'une concentration d'interleukine-2 humaine recombinante comprise entre 200 IU/mL et 2000 IU/mL, culture dans un environnement dont la température est comprise entre 36°C et 38°C et à humidité saturée de $CO_2$ de 5%, renouvellement par un milieu de culture frais tous les 2 à 3 jours et ajustement de la densité cellulaire de $0,5 \times 10^6$ à $5 \times 10^6$/ml, et culture durant 14 à 35 jours avant de récolter les cellules NK du sang ombilical.

2.  Un procédé d'expansion ex vivo selon la revendication 1, **caractérisé en ce que** les cellules mononucléaires du sang ombilical utilisées à l'étape 1) sont séparées du sang ombilical anticoagulant frais ou du sang ombilical congelé réanimé par:

    1.1: dilution du sang ombilical anticoagulant frais ou du sang ombilical congelé par tampon phosphate salin (PBS) selon un volume de 1 à 2 fois le volume de sang ombilical ;

    1.2: lent ajout du sang ombilical dilué ci-dessus à une solution de séparation lymphocytaire, et maintien d'une interface claire entre le sang ombilical dilué et la solution de séparation lymphocytaire, dans lequel le volume de sang ombilical dilué est égal au volume de solution de séparation lymphocytaire;

    1.3: centrifugation durant 20 à 30 minutes à température ambiante et à force centrifuge de 980 g; et

    1.4: après centrifugation, aspiration d'une couche mononucléaire membraneuse blanche, qui est la seconde couche depuis le haut, et lavage de la couche cellulaire mononucléaire par PBS, afin d'obtenir les cellules mononucléaires de sang ombilical.

Fig.1

Fig.2

Fresh Cord Blood     Frozen Resuscitation
Cord Blood

Fig.3

Fresh Cord Blood

Frozen Resuscitation Cord Blood

Fig.4

0 Day

1.23%    3.15%

77.53%

0.79%    1.50%

21 Days

94.58%    1.22%

1.03%

94.37%

Fresh Cord Blood

Frozen Resuscitation Cord Blood

Fig.5

0 Day

21 Days

Fresh Cord Blood          Frozen Resuscitation Cord Blood

Fig.6

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2011080740 A
- WO 2012009422 A
- US 2016075996 A
- CN 103146648 A
- CN 201310082166
- CN 102533650 A
- CN ZL201110456878

**Non-patent literature cited in the description**

- **KLINGEMANN H.** Challenges of cancer therapy with natural killer cells. *Cytotherapy,* 2015, vol. 17 (3), 245-249
- **KANG L ; VOSKINARIAN-BERSE V ; LAW E ; REDDIN T ; BHATIA M ; HARIRI A ; NING Y ; DONG D ; MAGUIRE T ; YARMUSH M et al.** Characterization and ex vivo Expansion of Human Placenta-Derived Natural Killer Cells for Cancer Immunotherapy. *Frontiers in immunology,* 2013, vol. 4, 101
- **SPANHOLTZ J ; PREIJERS F ; TORDOIR M ; TRILSBEEK C ; PAARDEKOOPER J ; DE WITTE T ; SCHAAP N ; DOLSTRA H.** Clinical-grade generation of active NK cells from cord blood hematopoietic progenitor cells for immunotherapy using a closed-system culture process. *PloS one,* 2011, vol. 6 (6), e20740
- **JAN SPANHOLTZ et al.** Clinical-grade Generation of active NK-cells from Cord Blood Hematopoietic Progenitor Cells for Immunotherapy Using a Closed-System Culture Process. *PLOS ONE,* 16 June 2011, vol. 6, e20740
- Adoptive immunotherapy with cytokine-induced killer cells generated with a new good manufacturing practice-grade protocol. *Cytotherapy,* 2012, 1-10